# EUROPEAN PATENT APPLICATION

(11) **EP 4 491 221 A1**
(43) Date of publication of application: **15.01.2025**
(21) Application number: 23888070.2
(22) Date of filing: 09.11.2023
(51) Int. Cl.: A61N 5/10

(54) **TREATMENT PLANNING SYSTEM, AUTOMATIC OVERLAP INSPECTION METHOD, AND METHOD FOR FORMULATING TREATMENT PLAN**

(30) Priority: 11.11.2022 CN 202211410819; 08.11.2023 CN 202311479375
(71) Applicant: Neuboron Therapy System Ltd., Xiamen, Fujian 361026 (CN)
(72) Inventor: ZHONG, Wanbing, Nanjing, Jiangsu 211112 (CN); CHEN, Jiang, Nanjing, Jiangsu 211112 (CN); TENG, Yi-chiao, Nanjing, Jiangsu 211112 (CN); JIANG, Bingxu, Nanjing, Jiangsu 211112 (CN)
(74) Representative: Gunzelmann, Rainer
(86) International application number: PCT/CN2023/130654
(87) International publication number: WO 2024/099385

(57) **Abstract**

The present invention relates to a treatment planning system, an automatic overlap inspection method, and a method for formulating a treatment plan. The treatment planning system comprises: an image processing module, for establishing an irradiated body model on the basis of medical image data, the irradiated body model comprising several voxel grids; a data processing module, for acquiring a beam source model and determining position parameters of the beam source model and the irradiated body model; an overlap detecting module, for selecting a reference object from the several voxel grids, determining whether the reference object overlaps with the beam source model according to a position relationship between the reference object and the beam source model, and adjusting the position parameters until the reference object does not overlap with the beam source model; and a treatment plan generating module, for generating a treatment plan on the basis of the adjusted position parameters. The present invention not only can automatically determine whether a collimator overlaps with the tissue of a patient and check whether a treatment plan will cause a collision of the collimator with the tissue of the patient during position arrangement, but also can correct a radiotherapy plan more quickly, and can automatically generate a corresponding treatment plan.

## Description

### TECHNICAL FIELD

The present disclosure relates to the technical field of treatment planning, and in particular to a treatment planning system, an automatic overlap checking method, and a method for formulating a treatment plan.

### BACKGROUND

The radiotherapy is a local treatment method with radioactive rays, and also an important method for treating cancers. With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma). In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam control, boron neutron capture therapy (BNCT) in neutron capture therapy serves as a better alternative to treat the cancers. Before the radiotherapy is administered, a treatment effect of the radiotherapy is estimated by the physicist to determine treatment procedures. This is the process for formulating a treatment plan.

When the treatment plan is formulated, it is crucial to determine an irradiating angle and an irradiating direction of the collimator relative to the patient. The outlet of the collimator is usually provided at a position close to the tumor site, for fear of excessive radiation damages to normal tissues. The collimator can guide radioactive particles to move toward the outlet, thereby emitting most of the radioactive particles to the tumor and protecting other normal tissues. In actual treatment, the tissues cannot overlap with the collimator in most cases, or else the patient is hardly set up to a position indicated by treatment parameters. If the collimator only overlaps with air in voxel grids, or the protruding treatment site on the surface of the body extends into the collimator, the treatment effect is not affected. When the treatment plan is formulated, whether the tissues overlap with the collimator is to be determined by the physicist. In case of an overlap in the actual treatment, for example, upon completion of dose calculation or even in setup, the treatment cannot be delivered smoothly.

### SUMMARY

In view of the above-mentioned technical problems, the present disclosure provides an efficient and accurate treatment planning system, an automatic overlap checking method for a treatment plan, and a method for formulating the treatment plan.

According to a first aspect, the present disclosure provides a treatment planning system, including:
an image processing module configured to acquire medical image data of an irradiated body, and establish a three-dimensional (3D) voxel model of the irradiated body on the basis of the medical image data, the 3D voxel model of the irradiated body including a plurality of voxel grids;
a data processing module configured to acquire a beam source model, and determine a positional parameter of the beam source model and a positional parameter of the 3D voxel model of the irradiated body;
an overlap detecting module configured to determine a positional relationship between the voxel grid and the beam source model; and
a treatment plan generating module configured to generate a treatment plan.

According to the treatment planning system provided by the present disclosure, the image processing module, the data processing module, the overlap detecting module, and the treatment plan generating module are provided to obtain the voxel grids of the 3D voxel model of the irradiated body, the positional parameter of the beam source model, and the positional parameter of the 3D voxel model of the irradiated body, etc. The overlap detecting module is provided to determine whether the 3D voxel model of the irradiated body and the beam source model overlap and are reasonable, thereby determining whether the collimator and the patient's tissue overlap and are reasonable under the treatment plan, and checking whether the treatment plan causes a collision between the collimator and the patient's tissue in setup. All procedures can be automatically checked and determined by the treatment planning system, thereby providing guidance for the physicist in advance to correct the treatment plan more quickly. Meanwhile, the treatment planning system can automatically generate the corresponding treatment plan.

In an embodiment, the overlap detecting module is configured to determine the positional relationship between the voxel grid and the beam source model on the basis of a positional relationship between a reference object and the beam source model; and the reference object is selected from the plurality of voxel grids.

In an embodiment, the overlap detecting module may be configured to determine a positional relationship between a reference object and the beam source model, as well as a positional relationship between the reference object and an internal irradiation space of the beam source model. When a treatment site of the irradiated body cannot extend into the internal irradiation space of the beam source model, a tissue of the irradiated body overlaps with neither the beam source model, nor the internal irradiation space of the beam source model. Meanwhile, when the beam source model is selected, whether the treatment site of the irradiated body can extend into the internal irradiation space of the beam source model is determined. The overlap detecting module determines whether the 3D voxel model of the irradiated body is allowed to extend into the internal irradiation space of the beam source model, and whether the 3D voxel model of the irradiated body extending into the internal irradiation space overlaps with the beam source model.

In an embodiment, the overlap detecting module may be configured to output an overlap alerting signal between the reference object and the beam source model. When the reference object overlaps with the beam source model, the overlap detecting module outputs the overlap alerting signal, so as to allow a user or a physicist to determine whether to adjust a relative positional relationship between the irradiated body and the beam source model. If yes, the relative positional relationship between the irradiated body and the beam source model may be adjusted according to the overlap alerting signal.

In an embodiment, the overlap detecting module may be configured to determine a tissue type of an overlapping reference object. When determining that the reference object overlaps with the beam source model, the overlap detecting module may determine whether the 3D voxel model of the irradiated body extending into the internal irradiation space of the beam source model overlaps with the beam source model, and further determine the tissue type of the overlapping reference object, thereby determining whether to adjust an overlapping tissue range.

In an embodiment, the overlap detecting module may be configured to determine an adjustment range of the positional parameter. When the treatment site of the irradiated body cannot extend into the internal irradiation space of the beam source model, after determining that the reference object overlaps with the beam source model, the system provides a reasonable adjustment range of the positional parameter for reference of the physicist. When the treatment site of the irradiated body can extend into the internal irradiation space of the beam source model, the overlap detecting module provides an adjustment range for an overlapping or non-overlapping positional parameter according to a tissue type of the reference object for reference of the physicist. This facilitates formulation of the treatment plan, and can further effectively improve the working efficiency of the physicist.

In an embodiment, the overlap detecting module may be configured to adjust the positional parameter. The overlap detecting module determines whether the reference object overlaps with the beam source model according to the positional relationship between the reference object and the beam source model, and automatically adjusts the positional parameter.

In an embodiment, the overlap detecting module may be configured to determine a type of the voxel grid.

In an embodiment, the type of the voxel grid includes a first-type grid and a second-type grid; the first-type grid is composed of a tissue of the irradiated body; the second-type grid is composed of air; and the reference object is selected from the first-type grid. When determining that the voxel grid is the first-type grid, the system selects the reference object in the grid or executes the subsequent overlap determining step or the overlap detecting module executes the subsequent overlap determining step. When determining that the voxel grid is the second-type grid, the system does not select the reference object in the grid and does not execute the subsequent determining step.

In an embodiment, the tissue type includes a first-type tissue and a second-type tissue; the first-type tissue is a superficial soft tissue; and the second-type tissue is a non-deformable tissue.

When the tissue type of the overlapping reference object is further determined, if the overlapping tissue type is the first-type tissue, namely the superficial tissue such as the skin or the superficial soft tissue such as the skin, the muscle, and the fat, whether to adjust the positional parameter is determined according to an overlapping range. If the overlapping tissue type is the second-type tissue, namely the non-deformable tissue such as the skeleton, a position of the irradiated body or a position of the beam source model is to be adjusted.

In an embodiment, the reference object may be selected from the second-type tissue.

When the tissue type of the overlapping reference object is the first-type tissue, the positional parameter of the 3D voxel model of the irradiated body or the positional parameter of the beam source model is not to be adjusted, or an adjustment range allowable to the positional parameter of the 3D voxel model of the irradiated body or the positional parameter of the beam source model is provided. When the tissue type of the overlapping reference object is the second-type tissue, the positional parameter of the 3D voxel model of the irradiated body or the positional parameter of the beam source model is to be adjusted, or a corresponding adjustment signal is provided for the physicist for reference.

In an embodiment, the reference object includes one, more or all of a vertex, a centroid, a random point, a profile or an outer surface of the voxel grid. The random point may be a random sampling point selected in the voxel grid. Enough random points are simulated to serve as the reference object.

In an embodiment, the positional parameter includes a relative distance between the beam source model and the 3D voxel model of the irradiated body, a relative angle, and a beam irradiating direction. The treatment planning system calculates and outputs the positional parameter according to the medical image data.

In an embodiment, the overlap alerting signal includes an overlap position, an overlap amplitude, and an overlap volume. When the reference object overlaps with the beam source model, the overlap alerting signal, including the overlap position, the overlap amplitude, and the overlap volume, is output for reference of the user or the physicist to adjust the relative positions.

In an embodiment, the beam source model is selected on the basis of the medical image data of the irradiated body. The data processing module can select the appropriate beam source model through the 3D voxel model of the irradiated body or the medical image data.

According to a second aspect, the present invention further provides an automatic overlap checking method, including:
a model acquiring step: acquiring a 3D voxel model of an irradiated body and a beam source model, the 3D voxel model of the irradiated body including a plurality of voxel grids;
a positional parameter acquiring step: acquiring a positional parameter of the beam source model and a positional parameter of the 3D voxel model of the irradiated body; and
an overlap determining step: determining a positional relationship between the 3D voxel model of the irradiated body and the beam source model on the basis of a positional relationship between the voxel grid and the beam source model.

In an embodiment, before the overlap determining step, the automatic overlap checking method further includes a reference object selecting step: selecting a reference object from the plurality of voxel grids; and in the overlap determining step, the positional relationship between the 3D voxel model of the irradiated body and the beam source model is determined on the basis of a positional relationship between the reference object and the beam source model.

In an embodiment, before the reference object selecting step or the overlap determining step or when the reference object selecting step or the overlap determining step is started, the automatic overlap checking method further includes a grid type determining step:
when determining that the voxel grid is a first-type grid, selecting the reference object in the grid and executing the overlap determining step; and when determining that the voxel grid is a second-type grid, not selecting the reference object in the grid and not executing the overlap determining step,
where a type of the voxel grid includes the first-type grid and the second-type grid; the first-type grid is composed of a tissue of the irradiated body; and the second-type grid is composed of air.

In an embodiment, the overlap determining step includes a position adjusting step: when the reference object overlaps with the beam source model, automatically adjusting the positional parameter of the beam source model or the positional parameter of the 3D voxel model of the irradiated body, until the reference object does not overlap with the beam source model.

In an embodiment, the overlap determining step further includes an overlap signal outputting step: when the reference object overlaps with the beam source model, outputting an overlap alerting signal.

In an embodiment, the overlap determining step further includes a space extension determining step: determining whether the 3D voxel model of the irradiated body extends into an internal irradiation space of the beam source model, and adjusting the positional parameter of the 3D voxel model of the irradiated body and the positional parameter of the beam source model.

In an embodiment, the space extension determining step includes a positional relationship determining step: determining a positional relationship between the reference object and the internal irradiation space of the beam source model.

In an embodiment, the positional relationship determining step may be executed in the model acquiring step; and when the beam source model is acquired, whether the irradiated body may extend into the internal irradiation space of the beam source model is determined according to a size of the internal irradiation space of the beam source model and a size of the irradiated body.

In an embodiment, the space extension determining step further includes a tissue type determining step: determining a tissue type of the irradiated body extending into the irradiation space, and adjusting the positional parameter according to the tissue type of the irradiated body.

In an embodiment, the tissue type in the tissue type determining step includes a first-type tissue and a second-type tissue; the first-type tissue is a superficial soft tissue; and the second-type tissue is a non-deformable tissue;
when the tissue type of the irradiated body is the first-type tissue, the positional parameter of the 3D voxel model of the irradiated body or the positional parameter of the beam source model is not adjusted, or an adjustment range for the positional parameter of the 3D voxel model of the irradiated body and an adjustment range for the positional parameter of the beam source model are provided; and
when the tissue type of the irradiated body is the second-type tissue, the positional parameter of the 3D voxel model of the irradiated body or the positional parameter of the beam source model is adjusted, or an adjustment signal is provided.

In an embodiment, the reference object is selected from the second-type tissue.

In an embodiment, the positional parameter in the positional parameter acquiring step includes a relative distance between the beam source model and the 3D voxel model of the irradiated body, a relative angle, and a beam irradiating direction.

In an embodiment, the reference object in the overlap determining step includes one, more or all of a vertex, a centroid, a random point, a profile or an outer surface of the voxel grid.

In an embodiment, the overlap alerting signal in the overlap signal outputting step includes an overlap position, an overlap amplitude, and an overlap volume.

In an embodiment, the beam source model in the model acquiring step is selected on the basis of medical image data of the irradiated body.

According to a third aspect, the present disclosure provides a method for formulating a treatment plan, including:
a model data acquiring step: acquiring medical image data of an irradiated body, and establishing a 3D voxel model of the irradiated body on the basis of the medical image data, the 3D voxel model of the irradiated body including a plurality of voxel grids;
a positional parameter determining step: determining a positional parameter of a beam source model and a positional parameter of the 3D voxel model of the irradiated body;
an overlap determining step: determining a positional relationship between the voxel grid and the beam source model and adjusting the positional parameter; and
a treatment plan generating step: generating a treatment plan.

In an embodiment, before the overlap determining step, the method for formulating a treatment plan further includes a reference object selecting step: selecting a reference object in the voxel grid; and in the overlap determining step, the positional relationship between the 3D voxel model of the irradiated body and the beam source model is determined on the basis of a positional relationship between the reference object and the beam source model.

In an embodiment, before the reference object selecting step or the overlap determining step or when the reference object selecting step or the overlap determining step is started, the method for formulating a treatment plan further includes a grid type determining step:
when determining that the voxel grid is a first-type grid, selecting the reference object in the grid and executing the overlap determining step; and when determining that the voxel grid is a second-type grid, not selecting the reference object in the grid and not executing the overlap determining step,
where a type of the voxel grid includes the first-type grid and the second-type grid; the first-type grid is composed of a tissue of the irradiated body; and the second-type grid is composed of air.

In an embodiment, the overlap determining step includes a position adjusting step: when the reference object overlaps with the beam source model, automatically adjusting the positional parameter of the beam source model or the positional parameter of the 3D voxel model of the irradiated body, until the reference object does not overlap with the beam source model.

In an embodiment, the overlap determining step further includes an overlap signal outputting step: when the reference object overlaps with the beam source model, outputting an overlap alerting signal.

In an embodiment, the overlap determining step further includes a space extension determining step: determining whether the 3D voxel model of the irradiated body extends into an internal irradiation space of the beam source model, and adjusting the positional parameter of the 3D voxel model of the irradiated body and the positional parameter of the beam source model.

In an embodiment, the space extension determining step includes a positional relationship determining step: determining a positional relationship between the reference object and the internal irradiation space of the beam source model.

In an embodiment, the positional relationship determining step may be executed in the positional parameter determining step; and when the beam source model is acquired, whether the irradiated body may extend into the internal irradiation space of the beam source model is determined according to a size of the internal irradiation space of the beam source model and a size of the irradiated body.

In an embodiment, the space extension determining step further includes a tissue type determining step: determining a tissue type of the irradiated body extending into the irradiation space, and adjusting the positional parameter according to the tissue type of the irradiated body.

In an embodiment, the tissue type in the tissue type determining step includes a first-type tissue and a second-type tissue; the first-type tissue is a superficial soft tissue; and the second-type tissue is a non-deformable tissue;
when the tissue type of the irradiated body is the first-type tissue, the positional parameter of the 3D voxel model of the irradiated body or the positional parameter of the beam source model is not adjusted, or an adjustment range for the positional parameter of the 3D voxel model of the irradiated body and an adjustment range for the positional parameter of the beam source model are provided; and
when the tissue type of the irradiated body is the second-type tissue, the positional parameter of the 3D voxel model of the irradiated body or the positional parameter of the beam source model is adjusted, or an adjustment signal is provided.

In an embodiment, the reference object is selected from the second-type tissue.

In an embodiment, the positional parameter in the positional parameter determining step includes a relative distance between the beam source model and the 3D voxel model of the irradiated body, a relative angle, and a beam irradiating direction.

In an embodiment, the reference object in the overlap determining step includes one, more or all of a vertex, a centroid, a random point, a profile or an outer surface of the voxel grid.

In an embodiment, the overlap alerting signal in the overlap signal outputting step includes an overlap position, an overlap amplitude, and an overlap volume.

In an embodiment, the beam source model in the model acquiring step is selected on the basis of the medical image data of the irradiated body.

According to the treatment planning system provided by the present disclosure, the image processing module, the data processing module, the overlap detecting module, and the treatment plan generating module are provided to obtain the voxel grids of the 3D voxel model of the irradiated body, the positional parameter of the beam source model, and the positional parameter of the 3D voxel model of the irradiated body, etc. The overlap detecting module is provided to determine whether the 3D voxel model of the irradiated body and the beam source model overlap and are reasonable, thereby determining whether the collimator and the patient's tissue overlap and are reasonable under the treatment plan, and checking whether the treatment plan causes a collision between the collimator and the patient's tissue in setup. All procedures can be automatically checked and determined by the treatment planning system, thereby providing guidance for the physicist in advance to correct the treatment plan more quickly. Meanwhile, the treatment planning system can automatically generate the corresponding treatment plan.

### BRIEF DESCRIPTION OF THE DRAWINGS

FIG. 1 is a schematic view of a treatment planning system according to an embodiment of the present disclosure;
FIG. 2 is a schematic view of a boron neutron capture reaction;
FIG. 3 illustrates an equation of a nuclear reaction in ¹⁰B(n,α)⁷Li neutron capture;
FIG. 4 is a block diagram of a neutron capture therapy system according to an embodiment of the present disclosure;
FIG. 5 is a flowchart of an automatic overlap checking method according to an embodiment of the present disclosure;

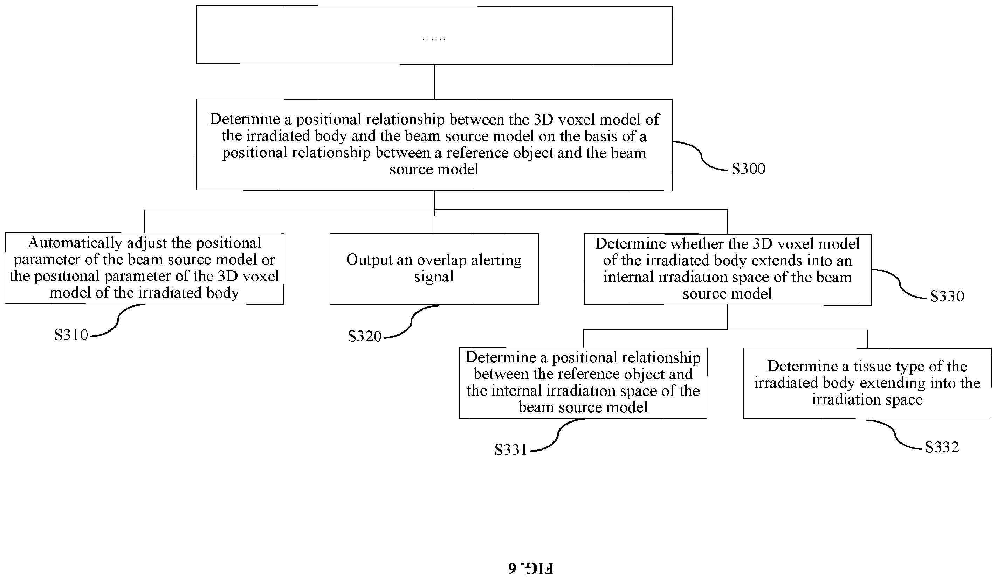

FIG. 6 is a flowchart of an automatic overlap checking method according to other embodiments of the present disclosure; and
FIG. 7 is a flowchart of a method for formulating a treatment plan according to an embodiment of the present disclosure.

In the figures:
100: BNCT device, 10: neutron beam source, 20: treatment planning system, 30: control system, 1: image processing module, 2: data processing module, 3: overlap detecting module, and 4: treatment plan generating module.

### DETAILED DESCRIPTION OF THE EMBODIMENTS

To make the objectives, technical solutions, and advantages of the present disclosure clearer, the present disclosure is further described in detail below with reference to the drawings and embodiments. It should be understood that the specific embodiments described herein are merely used to explain the present disclosure, rather than to limit the present disclosure.

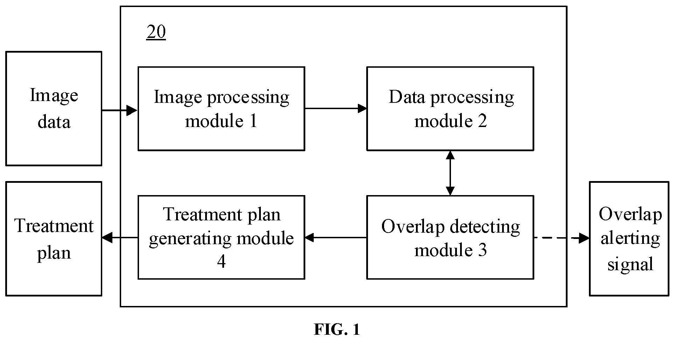

As shown in FIG. 1, the present disclosure provides a treatment planning system 20, which can determine whether an irradiated body overlaps with a beam source and generate a corresponding treatment plan.

As an implementation of the embodiment, the treatment plan is a treatment plan for administering radiotherapy, preferably a treatment plan for administering neutron capture therapy, and more preferably a treatment plan for administering BNCT. In an embodiment of the present disclosure, the BNCT is used as an example for brief description.

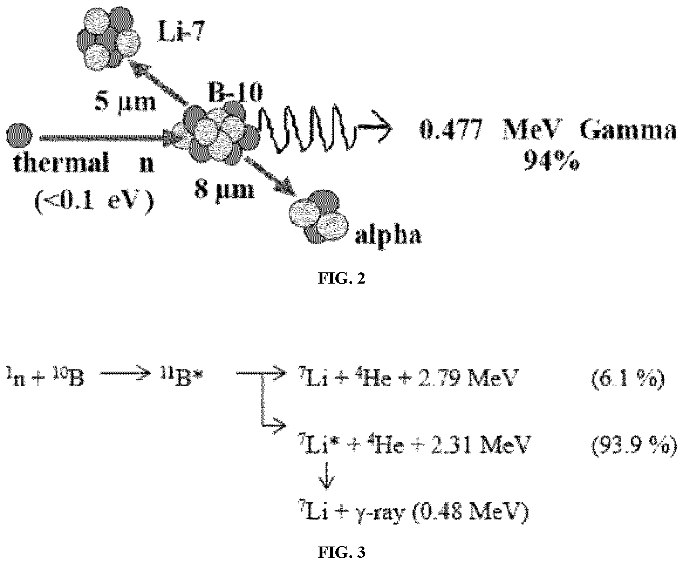

The neutron capture therapy, particularly the BNCT, has been applied increasingly in recent years as an effective method to treat the cancers. According to the BNCT, with a large capture cross section of the boron (¹⁰B)-containing drug for thermal neutrons, and through the ¹⁰B(n,α)⁷ Li neutron capture reaction and the nuclear fission reaction, ⁴He and ⁷Li heavy charged particles are generated. FIG. 2 and FIG. 3 respectively illustrate a schematic view of a boron neutron capture reaction and an equation of a nuclear reaction in ¹⁰B(n,α)⁷Li neutron capture. The two heavy charged particles have an average energy of about 2.33 MeV, and have characteristics of the high linear energy transfer (LET), and the short range. The alpha particle has the LET of 150 keV/µm, and the range of 8 µm. The ⁷Li heavy charged particle has the LET of 175 keV/µm, and the range of 5 µm. The total range of the two particles are approximately equivalent to a cell size, such that the radiation damage to an organism is limited to a cell level. When the boron-containing drug is aggregated to the tumor cells selectively, and an appropriate neutron source is provided, a purpose of locally killing the tumor cells on premise of no serious damage to normal tissues can be achieved.

Neutrons in the BNCT may be provided by a nuclear reactor or an accelerator. In an embodiment of the present disclosure, the accelerator is used as an example to provide the neutrons for the BNCT. The accelerator is configured to accelerate charged particles (such as photons and deuterons). The accelerated charged particles are interacted with a metal target to generate the neutrons. An appropriate nuclear reaction is selected according to characteristics such as a desired neutron yield and energy, available energies of the accelerated charged particles, a current, physical and chemical properties of the metal target, or the like. Nuclear reactions commonly discussed include ⁷Li(p,n)⁷Be and ⁹Be(p,n)⁹B, both of which are endothermic reactions. Energy thresholds of the two nuclear reactions are respectively 1.881 MeV and 2.055 MeV Epithermal neutrons with an energy level of keV are considered as an ideal neutron source in the BNCT. Theoretically, if photons with energies slightly higher than the threshold are used to bombard the lithium target, neutrons with relatively low energies can be generated, and can be used clinically without excessive moderation. However, the target made of the lithium (Li) and beryllium (Be) has a small action cross section with the photons at the threshold energy. In order to generate an enough large neutron flux, photons with high energies are usually selected to trigger the nuclear reaction.

The Monte Carlo method can accurately simulate collision trajectories and energy distributions of nuclear particles in a 3D space in the radiation object. In the BNCT, in order to simulate an absorbed dose of a human body under certain radiation conditions to facilitate formulation of the treatment plan, a medical image is processed repeatedly with a computer technology, so as to establish an accurate lattice model required by the Monte Carlo software, and perform simulation and calculation with the Monte Carlo software. The medical image data may be magnetic resonance imaging (MRI) data, computed tomography (CT) data, a positron emission tomography (PET) data, PET-CT data or X-ray imaging data. In the embodiment, description is made on the basis of the CT data. The CT data is often in a digital imaging and communications in medicine (DICOM) format. As is known to those skilled in the art, other medical image data may also be used in the modular treatment planning system and the system construction method in the present disclosure, provided that the medical image data can be converted into a 3D voxel prosthesis tissue model.

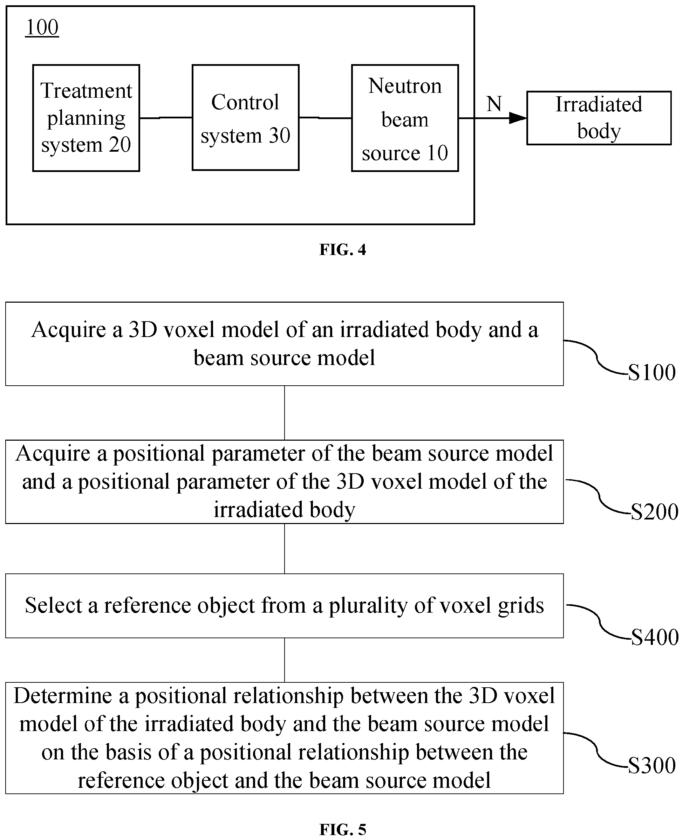

Referring to FIG. 4, a BNCT device 100 includes a neutron beam source 10, a treatment planning system 20, and a control system 30. The neutron beam source 10 includes a neutron generation device and a treatment table. The neutron generation device is configured to generate a neutron beam N for treatment and irradiate the neutron beam onto an irradiated site of a patient on the treatment table. The treatment planning system 20 is configured to generate a treatment plan according to medical image data of the patient. The control system 30 is configured to control the neutron beam source 10 on the basis of the treatment plan to perform irradiation for treatment. In an embodiment, a tissue model template library of the patient is stored in the treatment planning system 20. The treatment planning system 20 is configured to establish, according to the tissue model template library, a 3D voxel prosthesis tissue model corresponding to the medical image data of the irradiated site, perform simulation through a Monte Carlo simulation program on the basis of the 3D voxel prosthesis tissue model to calculate a dose distribution of the patient in the irradiation, and generate the treatment plan. The tissue model template library is preset. This prevents inaccurate model establishment and inaccurate dose calculation due to different experiences of the physicians and other operators, and does not define basic information of the organism in the lattice of the model with a lot of time and energy. The control system 30 is configured to acquire a corresponding treatment plan of a present patient from the treatment planning system 20, and control the neutron beam source 10 according to the treatment plan for irradiation.

The neutron generation device includes a neutron generation portion, a beam shaping assembly (BSA), and a collimator. The neutron generation portion includes an accelerator and a target. The accelerator is configured to accelerate charged particles (such as protons and deuterons) to generate a charged particle line such as a proton line. The charged particle line is irradiated onto the target and interacted with the target to generate a neutron line (a neutron beam). The target is preferably a metal target. An appropriate nuclear reaction is selected according to characteristics such as a desired neutron yield and energy, available energies of the accelerated charged particles, a current, physical and chemical properties of the metal target, or the like. Nuclear reactions commonly discussed include ⁷Li(p,n)⁷Be and ⁹Be(p,n)⁹B, both of which are endothermic reactions. In the embodiment of the present disclosure, the target is made of lithium. However, as is known to those skilled in the art, the target may also be made of a metal material other than the lithium and beryllium, such as tantalum (Ta) or tungsten (W). The target may be a circular plate, may also be other solid shapes, and may further be a liquid (liquid metal). The accelerator may be a linear accelerator, a cyclotron, a synchrotron and a synchrocyclotron. In other implementations, the neutron generation portion may be a nuclear reactor without the accelerator and the target.

No matter whether a neutron source in the BNCT comes from the nuclear reactor or the nuclear reaction between the accelerated charged particles and the target, a mixed radiation field is generated as a matter of fact, that is, the generated beam includes neutrons and photons having energies from low to high. As for the BNCT on deep-seated tumors, except the epithermal neutrons, the more the remaining radioactive rays, the greater the proportion causing non-selective dose deposition in the normal tissue. Therefore, the radioactive rays causing the unnecessary dose deposition should be reduced as much as possible. The BSA can adjust quality of the neutron beam generated by the neutron generation device, thereby reducing the unnecessary dose deposition. The collimator is configured to gather the neutron beam, such that the neutron beam has a high targeting ability in the treatment.

The BSA includes a reflector, a moderator, a thermal neutron absorber, a radiation shield, and a beam outlet. The moderator can adjust energies (>40 keV) of fast neutrons from the neutron generation device to an energy range (0.5 eV to 40 keV) of epithermal neutrons, and reduce thermal neutrons (<0.5 eV) as much as possible. The moderator is made of a material having a large action cross section with the fast neutrons but a small action cross section with the epithermal neutrons. As a preferred embodiment, the moderator is made of at least one of D₂O, AlF₃, FluentalTM, CaF₂, Li₂CO₃, MgF₂, and Al₂O₃. The reflector surrounds the moderator and reflects neutrons diffused through the moderator back to the neutron beam, thereby improving the utilization rate of the neutrons. The reflector is made of a material with a strong neutron reflectivity. As a preferred embodiment, the reflector is made of at least one of Pb or Ni. On a transmission path of the neutron beam, the thermal neutron absorber is provided behind the moderator, configured to absorb thermal neutrons passing through the moderator to reduce the thermal neutrons in the neutron beam, and made of a material having a large action cross section with the thermal neutrons. As a preferred embodiment, the thermal neutron absorber is made of Li⁻⁶. In other embodiments, because of the Li⁻⁶ in the material of the moderator, the thermal neutron absorber may not be provided individually, but the moderator serves as the thermal neutron absorber. The radiation shield is configured to shield neutrons and photons leaked from a portion other than the beam outlet. A material of the radiation shield includes at least one of a photon shielding material and a neutron shielding material. As a preferred embodiment, the material of the radiation shield includes lead (Pb) as the photon shielding material and polyethylene (PE) as the neutron shielding material.

The collimator is provided behind the beam outlet. An epithermal neutron beam from the collimator is irradiated onto the irradiated body. After passing through a superficial normal tissue of the irradiated body, the epithermal neutron beam is moderated as thermal neutrons to reach the tumor cells for treatment.

The treatment plan in the embodiment of the present disclosure includes an irradiation condition required by delivery of radiotherapy to the irradiated body. The irradiation condition includes a positional parameter, a dose parameter, etc. The positional parameter includes coordinate information capable of representing relative positions of a model of the irradiated body and a beam source model, various coordinate information or relative positional information of an irradiation source, and an angle or a direction indicated by the above relative positions. During formulation of the treatment plan, the positional parameter is calculated first before the dose calculation.

The beam source is usually located at an inlet of the collimator or an outlet of the collimator (at a side close to the irradiated body). In some cases, the irradiated body cannot extend into the collimator. In terms of calculation efficiency, the beam source at the outlet of the collimator is closer to the irradiated body, and with the beam source at the outlet of the collimator, the dose distribution can be calculated more quickly. In this case, the information of the beam source at the outlet is determined based on a condition where air is filled in the collimator. In order to formulate the reasonable treatment plan and calculate the accurate treatment dose, the irradiated body overlaps with neither the collimator, nor an internal space of the collimator. In other cases, the treatment site of the irradiated body can extent into the collimator, and the dose distribution is calculated with the beam source at the inlet of the collimator. In this case, the tissue of the irradiated body cannot overlap with the collimator. Conventionally, after the treatment plan is formulated by the user or the physicist, it is tedious in simulated treatment or actual treatment to observe or determine whether an overlap exists, or determine whether the overlap is reasonable.

In view of this, the treatment planning system 20 in the embodiment includes an image processing module 1, a data processing module 2, an overlap detecting module 3, and a treatment plan generating module 4.

The image processing module 1 is configured to acquire medical image data of an irradiated body. The medical image data is usually acquired through an external scanning device. The image processing module 1 is configured to establish a 3D voxel model of the irradiated body on the basis of the medical image data. The 3D voxel model of the irradiated body includes a plurality of voxel grids. The voxel grids for describing the 3D voxel model of the irradiated body are established by the image processing module 1.

The data processing module 2 is configured to acquire a beam source model, and determine a positional parameter of the beam source model and a positional parameter of the 3D voxel model of the irradiated body.

Further, the data processing module 2 is configured to acquire the 3D voxel model of the irradiated body from the image processing module 1, and acquire the beam source model from a preset library. The preset library may include beam source models of different shapes and different sizes, such that the data processing module 2 acquires the beam source model matching with the 3D voxel model of the irradiated body.

Further, the data processing module 2 can further be configured to select the appropriate beam source model through the 3D voxel model of the irradiated body or the medical image data. In other optional implementations, the 3D voxel model of the irradiated body may be input or selected by the user. The data processing module 2 may be configured to invoke the corresponding beam source model through input or selection of the user. The data processing module 2 is configured to calculate the positional parameter through the 3D voxel model of the irradiated body and the selected beam source model. The positional parameter includes a relative distance between the beam source model and the 3D voxel model of the irradiated body, a relative angle, and a beam irradiating direction. It is to be noted that the data processing module 2 can further be configured to calculate an irradiation parameter according to the image data or the 3D voxel model of the irradiated body. It may be understood that the collimator may not be provided in the present disclosure, and a beam from the beam outlet of the BSA is directly irradiated onto the irradiated body. For ease of description, when the collimator is provided, an outlet of the collimator is understood as the beam outlet. The device forming the beam outlet is called the beam source. The beam source model in the present disclosure is a model of the device forming the beam outlet.

Further, in other optional implementations, while the beam source model is selected, a position of the beam source is determined. That is, whether a treatment site of the irradiated body extends into the collimator when the treatment plan is formulated is selected.

Further, an irradiation space is formed at the beam outlet of the beam source. The irradiation space is surrounded by a periphery of the beam outlet. For example, the irradiation space may be a radial opening.

The overlap detecting module 3 is configured to determine a positional relationship between the voxel grid and the beam source model. In the embodiment, the overlap detecting module 3 is configured to determine the positional relationship between the voxel grid and the beam source model on the basis of a positional relationship between a reference object and the beam source model. The reference object is selected from at least one of the voxel grids. The reference object includes one, more or all of a vertex, a centroid, a random point, a profile or an outer surface of the voxel grid. Specifically, a type of the voxel grid includes a first-type grid and a second-type grid. The first-type grid is filled with a tissue of the irradiated body. The tissue of the first-type grid refers to an organic or inorganic component of the human body, such as an organ, a blood vessel, a skeleton, muscle, fat, a skin of the irradiated body. The second-type grid is filled with air. The reference object is selected from the first-type grid.

Further, the overlap detecting module 3 may be configured to determine whether a positional relationship between the 3D voxel model of the irradiated body and the beam source model is reasonable. Specifically, the overlap detecting module 3 or the data processing module 2 can determine whether the voxel grid is the first-type grid or the second-type grid. When it is determined that the voxel grid is the first-type grid, the reference object is selected in the grid, or a subsequent overlap determining step is executed, or the overlap detecting module 3 executes the subsequent overlap determining step. When it is determined that the voxel grid is the second-type grid, the reference object is not selected in the grid and the subsequent overlap determining step is not executed. The overlap detecting module 3 determines whether the reference object overlaps with the beam source model according to the positional relationship between the reference object and the beam source model, and adjusts the positional parameter.

Further, as an implementation, when determining that the reference object overlaps with the beam source model, the overlap detecting module 3 is configured to adjust a position of the 3D voxel model of the irradiated body or a position of the beam source model, and output a signal to the data processing module 2, such that the data processing module recalculates the positional parameter, acquires a new positional parameter and determines an overlap again, until the reference object does not overlap with the beam source model.

Further, as another implementation, when determining that the reference object overlaps with the beam source model, the overlap detecting module 3 is configured to output a signal to the data processing module 2. The data processing module 2 is configured to adjust and recalculate the positional parameter of the 3D voxel model of the irradiated body or the positional parameter of the beam source model. The overlap detecting module 3 is configured to acquire a new positional parameter, and determine an overlap again, until the reference object does not overlap with the beam source model.

It is to be noted that the above embodiment is based on a case where the treatment site of the irradiated body cannot extend into the collimator. That is, the tissue of the irradiated body overlaps with neither the beam source model, nor the internal irradiation space of the beam source model.

Further, in other optional implementations, the overlap detecting module 3 may further be configured to determine whether a positional relationship between the 3D voxel model of the irradiated body and the internal irradiation space of the beam source model is reasonable, namely whether the 3D voxel model of the irradiated body is allowed to extend into the internal irradiation space of the beam source model, whether the 3D voxel model of the irradiated body extending into the internal irradiation space of the beam source model overlaps with the beam source model, and whether the treatment site of the irradiated body may extend into the internal irradiation space of the beam source model when the beam source model is selected. When determining that the reference object overlaps with the beam source model, the overlap detecting module 3 can determine whether the 3D voxel model of the irradiated body extending into the internal irradiation space of the beam source overlaps with the beam source model.

Further, the overlap detecting module 3 may further be configured to determine a tissue type of an overlapping reference object. The tissue type includes a first-type tissue and a second-type tissue. The first-type tissue is a superficial tissue such as the skin or a superficial soft tissue such as the skin, the muscle, and the fat. The second-type tissue is a non-deformable tissue such as the skeleton. The reference object is selected from the second-type tissue. Specifically, if the tissue type of the overlapping reference object is the first-type tissue, whether to adjust the positional parameter is determined according to an overlapping range or an adjustment range of the positional parameter is provided by the treatment planning system 20. If the tissue type of the overlapping reference object is the second-type tissue, the position of the irradiated body or the position of the beam source model is to be adjusted. After the adjustment, the data processing module 2 calculates the positional parameter, and the overlap detecting module 3 determines the positional parameter, or the overlap detecting module 3 directly determines the positional parameter. When the reference object and the beam source model are reasonable, a positional parameter by this time is output to the treatment plan generating module 4 for dose calculation, thereby formulating the treatment plan.

Further, in another implementation, the overlap detecting module 3 may further be configured to output an overlap alerting signal between the reference object and the beam source, so as to allow the user or the physicist to determine whether to adjust the relative positional relationship between the irradiated body and the beam source model. If yes, after calculation of the data processing module 2, the overlap detecting module 3 redetermines the relative positional relationship, or the overlap detecting module 3 directly determines the relative positional relationship. Specifically, the overlap alerting signal in the embodiment includes an overlap position, an overlap amplitude, and an overlap volume. When the positional relationship between the reference object and the beam source model is reasonable, a positional parameter by this time is output to the treatment plan generating module 4 for dose calculation, thereby formulating the treatment plan.

As shown in FIG. 5, an embodiment of the present disclosure further provides an automatic overlap checking method based on a treatment plan. The automatic overlap checking method includes the following steps:
A model acquiring step S100: A 3D voxel model of an irradiated body and a beam source model are acquired, the 3D voxel model of the irradiated body including a plurality of voxel grids.

A positional parameter acquiring step S200: A positional parameter of the beam source model and a positional parameter of the 3D voxel model of the irradiated body are acquired.

An overlap determining step S300: A positional relationship between the 3D voxel model of the irradiated body and the beam source model is determined on the basis of a positional relationship between the voxel grid and the beam source model.

Before the overlap determining step, the method for formulating a treatment plan further includes a reference object selecting step S400: A reference object is selected from the plurality of voxel grids. In the overlap determining step S300, the positional relationship between the 3D voxel model of the irradiated body and the beam source model is determined on the basis of a positional relationship between the reference object and the beam source model.

In the model acquiring step S100, the acquired beam source model is preset or guided to a treatment planning system 20 through other devices. Specifically, there are a plurality of beam source models that are prestored in the treatment planning system 20, and can be selected by a doctor on the basis of medical image data of the irradiated body. The 3D voxel model of the irradiated body is established on the basis of the medical image data of the irradiated body. The 3D voxel model of the irradiated body includes the plurality of voxel grids.

In the positional parameter acquiring step S200, the positional parameter of the beam source model and the positional parameter of the 3D voxel model of the irradiated body each include at least a relative distance between the beam source model and the 3D voxel model of the irradiated body, a relative angle, and a beam irradiating direction. The treatment planning system 20 calculates and outputs the positional parameter according to the medical image data.

Before the reference object selecting step S400 or the overlap determining step S300 or when the reference object selecting step S400 or the overlap determining step S300 is started, the method for formulating a treatment plan further includes a grid type determining step S500:

When it is determined that the voxel grid is a first-type grid, the reference object is selected in the grid and the overlap determining step is executed. When it is determined that the voxel grid is a second-type grid, the reference object is not selected in the grid and the overlap determining step is not executed.

A type of the voxel grid includes the first-type grid and the second-type grid. The first-type grid is composed of a tissue of the irradiated body. The second-type grid is composed of air. Specifically, the tissue of the first-type grid refers to an organic or inorganic component of the human body, such as an organ, a blood vessel, a skeleton, muscle, fat, a skin of the irradiated body. In the reference object selecting step S400, the reference object is selected from the first-type grid. The voxel grid is usually a polyhedron. In the embodiment, the voxel grid is a hexahedron. The reference object is a point, a line or a surface of the voxel grid. In order to simplify the calculation process and reduce occupation of the calculation in the system memory, the point of the voxel grid is preferably taken as the reference object. In the embodiment, the reference object is a vertex of the voxel grid. In a preferred implementation, whether eight vertexes of the first voxel grid overlap with the beam source model is determined. If any one of the vertexes overlaps with the beam source model, it is determined that the 3D voxel model of the irradiated body overlaps with the beam source model. In other embodiments, the reference object includes, but is not limited to, one, more or all of a centroid, a random point, a profile or an outer surface of the voxel grid. For example, the random point may be a random sampling point selected in the voxel grid. Enough random points are simulated to serve as the reference object.

Further, the overlap determining step S300 includes a position adjusting step S310: When the reference object overlaps with the beam source model, the positional parameter of the beam source model or the positional parameter of the 3D voxel model of the irradiated body is automatically adjusted, until the reference object does not overlap with the beam source model.

Further, in order to simplify the calculation process and reduce occupation of the calculation in the system memory, when the positional parameter of the beam source model or the positional parameter of the 3D voxel model of the irradiated body is adjusted, only relative positions of the beam source model and the 3D voxel model of the irradiated body, but not the beam irradiating direction, may be adjusted. That is, the relative positions of the beam source model and the 3D voxel model of the irradiated body are translated along the beam irradiating direction, so as to minimize influences on other parameters. All procedures can be automatically determined and adjusted with the treatment planning system 20. This makes the output treatment plan more accurate, and prevents the inappropriate actual setup.

Further, the overlap determining step S300 further includes an overlap signal outputting step S320: When the reference object overlaps with the beam source model, an overlap alerting signal is output, so as to allow a user or a physicist to determine whether to adjust a relative positional relationship between the irradiated body and the beam source model. If yes, the relative positional relationship between the irradiated body and the beam source model is adjusted according to the overlap alerting signal. After the relative positional relationship between the irradiated body and the beam source model is adjusted, the overlap determining step S300 is repeated. Further, the overlap alerting signal may include an overlap position, an overlap amplitude, and an overlap volume for reference of the user or the physicist to adjust the relative positions.

Further, the overlap determining step S300 further includes a space extension determining step S330: Whether the 3D voxel model of the irradiated body extends into an internal irradiation space of the beam source model is determined, and the positional parameter of the 3D voxel model of the irradiated body and the positional parameter of the beam source model are adjusted.

Further, the space extension determining step S330 may further include the following steps:
A positional relationship determining step S331: A positional relationship between the reference object and the internal irradiation space of the beam source model is determined. Similar to the overlap determining manner in the step S300 and the step S400, whether the 3D voxel model of the irradiated body is allowed to extend into the internal irradiation space of the beam source model, as well as whether 3D voxel model of the irradiated body extends into the irradiation space, is determined by selecting the reference object and determining the positional relationship of the reference object. Further, the positional relationship determining step S331 in the embodiment may also be executed in the model acquiring step S100. When the beam source model is selected, whether the irradiated body may extend into the internal irradiation space, particularly whether a treatment site of the irradiated body may extend into the internal irradiation space, is determined according to a size of the internal irradiation space of the beam source model and a size of the irradiated body.

A tissue type determining step S332: A tissue type of the irradiated body extending into the irradiation space is determined, and the positional parameter is adjusted according to the tissue type of the irradiated body. In the embodiment, the tissue type of the irradiated body is determined by determining a type of the voxel grid or the reference object. Specifically, the tissue type includes a first-type tissue and a second-type tissue. The first-type tissue is a superficial soft tissue, including the skin, the muscle, and the fat. The second-type tissue is non-deformable tissue such as the skeleton. Further, when the tissue type of the irradiated body is the first-type tissue, the positional parameter of the 3D voxel model of the irradiated body or the positional parameter of the beam source model is not to be adjusted, or an adjustment range for the positional parameter of the 3D voxel model of the irradiated body and an adjustment range for the positional parameter for the positional parameter of the beam source model are provided. When the tissue type of the irradiated body is the second-type tissue, the positional parameter of the 3D voxel model of the irradiated body or the positional parameter of the beam source model is to be adjusted, or an adjustment signal is provided. The reference object may be selected from the second-type tissue.

Specifically, the positional relationship determining step S331 and the tissue type determining step S332 are not limited in sequential order. The type of the irradiated body may be determined first and then the reference object is selected. The reference object may also be selected from tissues of all types, and then a positional relationship of the desired reference object is determined.

A treatment plan is generated by a treatment plan generating module 4 on the basis of an adjusted positional parameter. In case of an overlap, when the positional parameter is readjusted, an irradiation parameter is recalculated by the treatment plan generating module 4 on the basis of an irradiation parameter obtained by a data processing module 2 and an adjusted unknown parameter. A generated treatment plan includes an adjusted positional parameter and a new irradiation parameter. Further, when the positional parameter is adjusted, the relative positions of the 3D voxel model of the irradiated body and the beam source model are mainly adjusted. Without changing a direction parameter, only a dose parameter may be recalculated when the irradiation parameter is recalculated.

The modules of the treatment planning system 20 may be implemented in whole or in part by software, hardware, or any combination thereof. The modules may be embedded in or independent of a processor of a computer device in a form of hardware, or stored in a memory of the computer device in a form of software, such that the processor can easily invoke and execute corresponding operations of the modules.

The treatment planning system 200 may further include a terminal, a communication module, a server, a data storage module, etc. The terminal communicates with the server through the communication module. The image processing module 1, the data processing module 2, the overlap detecting module 3, and the treatment plan generating module 4 may be integrated into the terminal. The terminal may be, but is not limited to, various personal computers, notebook computers, smartphones, tablet computers, Internet of Things (IoT) devices, and portable wearable devices. The IoT devices may be smart speakers, smart televisions, smart air conditioners, smart vehicle-mounted devices, etc. The portable wearable devices may be smart watches, smart bracelets, headset devices, etc. The data storage module may be integrated onto the server 1, and may also be provided on a cloud or other network servers. The data storage module includes a non-volatile storage medium and an internal memory. The non-volatile storage medium stores programs and data involved in the image processing module 1, the data processing module 2, the overlap detecting module 3, and the treatment plan generating module 4, and stores operation systems and computer programs. The internal memory provides an environment for operation of the operating systems and the computer programs in the nonvolatile storage medium. The server can be implemented as a standalone server or a server cluster consisting of a plurality of servers. The communication interface is configured for wired or wireless communication with an external terminal. Wireless communication can be achieved through wireless fidelity (WiFi), service provider networks, near-field communication (NFC) or other means.

The terminal further includes a display device and an input device. The image processing module 1, the data processing module 2, the overlap detecting module 3, and the treatment plan generating module 4 may show corresponding data and interface through the display device. The image processing module 1, the data processing module 2, the overlap detecting module 3, and the treatment plan generating module 4 may acquire, through the input device, a parameter input by the user. The display device may be a display screen particularly. The display screen may be a liquid crystal display (LCD) screen or an e-ink display screen. The input device may be a touch layer covering the display screen, may also be a key, a trackball or a touchpad set on the terminal, and may further be an external keyboard, a touchpad or a mouse, etc.

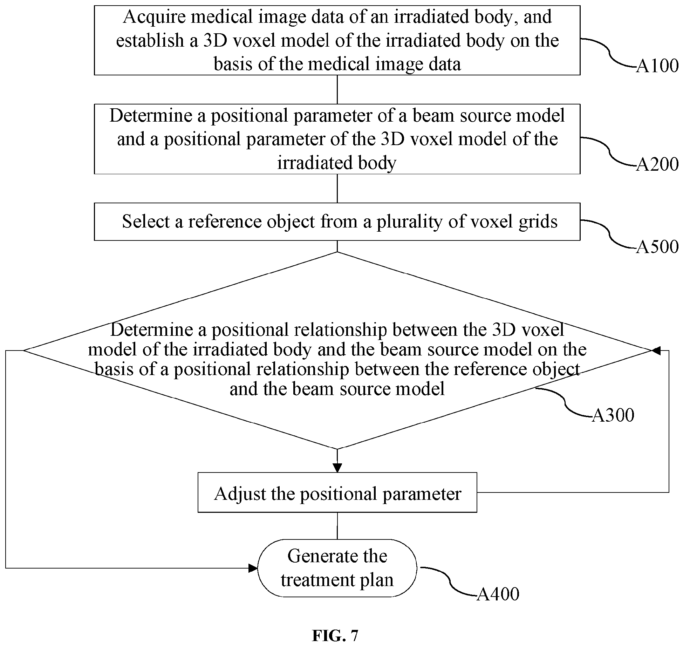

As shown in FIG. 7, an embodiment of the present disclosure further includes a method for formulating a treatment plan. As a manner for operating a treatment planning system 20, the method for formulating a treatment plan can determine an overlap and generate a corresponding treatment plan. Parts same as or similar to the above contents are not repeated. The method for formulating a treatment plan includes the following steps:
A model data acquiring step A100: Medical image data of an irradiated body is acquired, and a 3D voxel model of the irradiated body is established on the basis of the medical image data, the 3D voxel model of the irradiated body including a plurality of voxel grids.

A positional parameter determining step A200: A positional parameter of a beam source model and a positional parameter of the 3D voxel model of the irradiated body are determined.

An overlap determining step A300: A positional relationship between the voxel grid and the beam source model is determined and the positional parameter is adjusted.

A treatment plan generating step A400: The treatment plan is generated.

Further, before the overlap determining step A300 in the embodiment, the method for formulating a treatment plan further includes a reference object selecting step A500: A reference object is selected from the plurality of voxel grids. In the overlap determining step A300, the positional relationship between the 3D voxel model of the irradiated body and the beam source model is determined on the basis of a positional relationship between the reference object and the beam source model.

Further, the reference object selecting step A500 in the embodiment further includes:
A501: Whether the voxel grid is a first-type grid or a second-type grid is determined.
A502: The reference object is selected in the first-type grid.

Further, the overlap determining step A300 in the embodiment further includes:
A301: Whether the reference object overlaps with the beam source model is determined, or whether the reference object extends into the beam source model is determined.
A302: When the reference object overlaps with the beam source model or extends into the beam source model, the positional parameter is adjusted, and the step A301 is repeated. When the reference object does not overlap with the beam source model or does not extend into the beam source model, the step A400 is executed. When the reference object does not overlap with the beam source from the beginning, the generated treatment plan includes the positional parameter determined in the step A200.

It should be understood that although the steps in the flowcharts in the above embodiments are shown in sequence as indicated by the serial numbers, arrows or connecting lines, these steps are not necessarily performed in sequence as indicated by the serial numbers. The execution order of these steps is not strictly limited, and these steps may be executed in other orders, unless clearly described otherwise. Moreover, at least some of the steps in the flowcharts in the above embodiments may include a plurality of steps or stages. The steps or stages are unnecessarily executed at the same time, but may be executed at different times. The execution order of the steps or stages is unnecessarily carried out sequentially, but may be executed alternately with other steps or at least some of the steps or stages of other steps.

Those of ordinary skill in the art may understand that all or some of the procedures in the method of the foregoing embodiments may be implemented by a computer program instructing related hardware. The computer program may be stored in a nonvolatile computer-readable storage medium. When the computer program is executed, the procedures in the embodiments of the foregoing method may be performed. Any reference to a memory, a storage, a database, or other media used in the embodiments of the present application may include a non-volatile and/or volatile memory. The non-volatile memory may include a read-only memory (ROM), a magnetic tape, a floppy disk, a flash memory, an optical memory, a high-density embedded non-volatile memory, a Resistive Random Access Memory (ReRAM), a Magnetoresistive Random Access Memory (MRAM), a Ferroelectric Random Access Memory (FRAM), a Phase Change Memory (PCM), a graphene memory, and the like. The volatile memory may include a random access memory (RAM) or an external cache memory. As an illustration rather than a limitation, the RAM may be in various forms, such as a static random access memory (SRAM) or a dynamic random access memory (DRAM). The database in the embodiments of the present disclosure may include at least one of a relational database and a non-relational database. The non-relational database may include a distributed database based on a blockchain, but is not limited thereto. The processor in the embodiments of the present disclosure may be a general processor, a central processor, a graphics processor, a digital signal processor (DSP), a programmable logic device, and a data processing logic device based on quantum computing, but is not limited thereto.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

The above embodiments are merely illustrative of several implementations of the present disclosure, and the description thereof is more specific and detailed, but is not to be construed as a limitation to the patentable scope of the present disclosure. It should be noted that those of ordinary skill in the art can further make variations and improvements without departing from the conception of the present disclosure. These variations and improvements all fall within the protection scope of the present disclosure. Therefore, the protection scope of the present disclosure should be defined by the appended claims.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

The above embodiments are merely illustrative of several implementations of the present disclosure, and the description thereof is more specific and detailed, but is not to be construed as a limitation to the patentable scope of the present disclosure. It should be noted that those of ordinary skill in the art can further make variations and improvements without departing from the conception of the present disclosure. These variations and improvements all fall within the protection scope of the present disclosure. Therefore, the protection scope of the present disclosure should be defined by the appended claims.

## Claims

1. Atreatment planning system, comprising:
an image processing module configured to acquire medical image data of an irradiated body, and establish a three-dimensional (3D) voxel model of the irradiated body on the basis of the medical image data, the 3D voxel model of the irradiated body comprising a plurality of voxel grids;
a data processing module configured to acquire a beam source model, and determine a positional parameter of the beam source model and a positional parameter of the 3D voxel model of the irradiated body;
an overlap detecting module configured to determine a positional relationship between the voxel grid and the beam source model; and
a treatment plan generating module configured to generate a treatment plan.

2. The treatment planning system according to claim 1, wherein the overlap detecting module is configured to determine the positional relationship between the voxel grid and the beam source model on the basis of a positional relationship between a reference object and the beam source model; and the reference object is selected from the plurality of voxel grids.

3. The treatment planning system according to claim 1, wherein the overlap detecting module is configured to determine a positional relationship between a reference object and the beam source model, as well as a positional relationship between the reference object and an internal irradiation space of the beam source model.

4. The treatment planning system according to claim 2, wherein the overlap detecting module is configured to determine a type of the voxel grid.

5. The treatment planning system according to claim 4, wherein the type of the voxel grid comprises a first-type grid and a second-type grid; the first-type grid is composed of a tissue of the irradiated body; the second-type grid is composed of air; and the reference object is selected from the first-type grid.

6. The treatment planning system according to claim 2, wherein the overlap detecting module is configured to determine a tissue type of an overlapping reference object.

7. The treatment planning system according to claim 6, wherein the tissue type comprises a first-type tissue and a second-type tissue; the first-type tissue is a superficial soft tissue; and the second-type tissue is a non-deformable tissue.

8. The treatment planning system according to claim 7, wherein the reference object is selected from the second-type tissue.

9. The treatment planning system according to claim 2, wherein the reference object comprises one, more or all of a vertex, a centroid, a random point, a profile or an outer surface of the voxel grid.

10. The treatment planning system according to claim 1, wherein the positional parameter comprises a relative distance between the beam source model and the 3D voxel model of the irradiated body, a relative angle, and a beam irradiating direction.

11. An automatic overlap checking method, comprising:
a model acquiring step: acquiring a three-dimensional (3D) voxel model of an irradiated body and a beam source model, the 3D voxel model of the irradiated body comprising a plurality of voxel grids;
a positional parameter acquiring step: acquiring a positional parameter of the beam source model and a positional parameter of the 3D voxel model of the irradiated body; and
an overlap determining step: determining a positional relationship between the 3D voxel model of the irradiated body and the beam source model on the basis of a positional relationship between the voxel grid and the beam source model.

12. The automatic overlap checking method according to claim 11, before the overlap determining step, further comprising a reference object selecting step: selecting a reference object from the plurality of voxel grids, wherein in the overlap determining step, the positional relationship between the 3D voxel model of the irradiated body and the beam source model is determined on the basis of a positional relationship between the reference object and the beam source model.

13. The automatic overlap checking method according to claim 12, before the reference object selecting step or the overlap determining step or when the reference object selecting step or the overlap determining step is started, further comprising a grid type determining step:
when determining that the voxel grid is a first-type grid, selecting the reference object in the grid and executing the overlap determining step; and when determining that the voxel grid is a second-type grid, not selecting the reference object in the grid and not executing the overlap determining step,
wherein a type of the voxel grid comprises the first-type grid and the second-type grid; the first-type grid is composed of a tissue of the irradiated body; and the second-type grid is composed of air.

14. The automatic overlap checking method according to claim 12, wherein the overlap determining step comprises a position adjusting step: when the reference object overlaps with the beam source model, automatically adjusting the positional parameter of the beam source model or the positional parameter of the 3D voxel model of the irradiated body, until the reference object does not overlap with the beam source model.

15. The automatic overlap checking method according to claim 12, wherein the overlap determining step further comprises a space extension determining step: determining whether the 3D voxel model of the irradiated body extends into an internal irradiation space of the beam source model.

16. A method for formulating a treatment plan, comprising:
a model data acquiring step: acquiring medical image data of an irradiated body, and establishing a three-dimensional (3D) voxel model of the irradiated body on the basis of the medical image data, the 3D voxel model of the irradiated body comprising a plurality of voxel grids;
a positional parameter determining step: determining a positional parameter of a beam source model and a positional parameter of the 3D voxel model of the irradiated body;
an overlap determining step: determining a positional relationship between the voxel grid and the beam source model and adjusting the positional parameter; and
a treatment plan generating step: generating a treatment plan.
